# EUROPEAN PATENT APPLICATION

(11) **EP 4 481 036 A1**
(43) Date of publication of application: **25.12.2024**
(21) Application number: 23756253.3
(22) Date of filing: 08.02.2023
(51) Int. Cl.: C12N 5/10, A61K 31/568, A61K 35/545, A61P 15/00, A61P 15/10, C12N 5/02, C12N 5/071

(54) **METHOD FOR PREPARING HUMAN PLURIPOTENT STEM CELL-DERIVED LEYDIG-LIKE CELLS, AND HUMAN PLURIPOTENT STEM CELL-DERIVED LEYDIG-LIKE CELL POPULATION**

(30) Priority: 15.02.2022 JP 2022021533
(71) Applicant: National University Corporation Kobe University, Kobe-shi, Hyogo 657-8501 (JP)
(72) Inventor: AOI, Takashi, Kobe-shi, Hyogo 657-8501 (JP); SATO, Katsuya, Kobe-shi, Hyogo 657-8501 (JP); FUJISAWA, Masato, Kobe-shi, Hyogo 657-8501 (JP)
(74) Representative: Krauss, Jan
(86) International application number: PCT/JP2023/004081
(87) International publication number: WO 2023/157727

(57) **Abstract**

Provided are a method of producing a human pluripotent stem cell-derived Leydig-like cell (Leydig-like cells) capable of secreting testosterone stably and persistently, and a human pluripotent stem cell-derived Leydig-like cell. Also provided is a human pluripotent stem cell-derived Leydig-like cell that enables long-term maintenance of a Leydig-like cell secreting a sufficient amount of testosterone, and is improved in efficiency of differentiation induction. The method includes the steps of: culturing a human pluripotent stem cell under a culture condition with addition of one or a plurality of kinds selected from the group consisting of: a WNT canonical pathway activator; bone morphogenetic protein 4 (BMP4); and vascular endothelial growth factor (VEGF); and forcedly expressing NR5A1, and is achieved by controlling the timing of addition and removal of cyclic adenosine monophosphate (cAMP), suspension culture, adhesion culture, and the like.

## Description

### Technical Field

The present invention relates to a method of producing a human pluripotent stem cell-derived Leydig-like cell, and a human pluripotent stem cell-derived Leydig-like cell population.

This application claims priority based on Japanese Patent Application No. 2022-021533, which is incorporated herein by reference.

### Background Art

In recent years, age-related male hypogonadal syndrome (late-onset hypogonadism (LOH) syndrome), which is caused by an age-related decrease in male hormone and develops various symptoms, has attracted attention. To deal with this, hormone replacement therapies of testosterone have been performed. However, hormone replacement therapies result in a hormone value different from that of a physiological secretion pattern and have a short duration of action, thus requiring repeated dosing and imposing a heavy burden on a patient. There has been a demand for development of a novel therapeutic method that needs no such repeated dosing, is improved in duration for hospital visit, labor, pain associated with injection, and the like, and leads to an improvement in quality of life (QOL).

The hypothalamus produces gonadotropin-releasing hormone (GnRH). In response to production of GnRH, the anterior pituitary gland produces luteinizing hormone (LH) and follicle-stimulating hormone (FSH). Leydig cells in the testis are estimated to produce from 5 mg/day to 10 mg/day of testosterone in response to LH. Testosterone is synthesized from cholesterol via several kinds of intermediate compounds, such as dehydroepiandrosterone (DHEA) and androstenedione.

There have been attempted to develop methods of inducing differentiation into various kinds of cells using pluripotent stem cells, which have self-replicability and multilineage potential. Techniques employing embryonic stem cells (ES cells) and induced pluripotent stem cells (iPS cells) among the pluripotent stem cells have attracted attention. Various attempts have been made to induce differentiation into steroid hormone-producing cells of the testis and the adrenal gland, by stably introducing steroidogenic factor-1 (SF-1) being a transcription factor into various mesenchymal stem cells, and adding cyclic adenosine monophosphate (cAMP) to a medium. SF-1 is a member of the nuclear receptor family of intracellular transcription factors and is encoded by a nuclear receptor subfamily 5, group A, member 1 (NR5A1) gene.

One method has been reported in which SF-1 is introduced into two types of human ES cells (H9 strain and KhES 1 strain) and one type of human iPS cell line (201B7 strain) to induce differentiation into steroid hormone-producing cells. In this method, each of the human ES cell lines and the human iPS cell line is induced to differentiate into mesodermal cells through use of 6-bromoindirubin-3'-oxime (BIO) and a glycogen synthase kinase 3 (GSK-3) inhibitor, and the mesodermal cells are sorted with flow cytometry, followed by introduction of SF-1 into the mesodermal cells with a pCMFlag-hsNR5A1-containing plasmid for expression of SF-1, leading to expression. The resulting cells are further cultured in a medium containing 8-bromo-cAMP (8-Br-cAMP) and induced to differentiate into steroid hormone-producing cells. However, the report refers to production of progesterone and cortisol, but does not describe production of testosterone and does not disclose expression of markers that are selective indicators for a Leydig cell (INSL3, 17βHSD3, LHCGR, and the like) (Non Patent Literature 1).

Another method is disclosed in which murine pluripotent stem cells (ES cells) are induced to differentiate into mesenchymal stem cells that then undergo conditional expression of SF-1 to differentiate into steroid hormone-producing cells (Patent Literature 1). However, the disclosure does not report production of hormones, such as testosterone and cortisol, and does not show expression of markers that are indicators for a Leydig cell.

One report describes that murine SF-1 was introduced into murine ES cells (OriCellStrain C57BL/6 mESCs) with a lentivirus vector to produce SF-1⁺ESC strain (ESC-SF-1), which was then further cultured in a medium containing 8-Br-cAMP and forskolin and induced to differentiate into progenitor Leydig cells (PLCs). The description shows that treatment of ESC-SF-1 with 8-Br-cAMP and forskolin induced differentiation into PLCs more effectively than treatment with only 8-Br-cAMP. In this report, the PLCs thus obtained were then transplanted as Leydig-like cells into rats, followed by measurement of markers and the like that are indicators for a Leydig cell from serum samples and the like (Non Patent Literature 2).

One disclosure provides a method in which NR5A1 is expressed in human pluripotent stem cells to create human pluripotent stem cell-derived Leydig-like cells that express INSL3 and also express at least one marker selected from 17βHSD3 and LHCGR (Patent Literature 2). In addition, another disclosure shows a method of creating human pluripotent stem cell-derived Leydig-like cells that express HSD17B3, INSL3, LHCGR, and the like (Non-Patent Literature 3). However, there has been need for production of human pluripotent stem cell-derived Leydig-like cells that can persistently secrete testosterone more stably.

### Citation List

### Non Patent Literature

[NPL 1] Endocrinology, 153(9): 4336-4345 (2012)
[NPL 2] STEM CELLS AND DEVELOPMENT, 24(4): 459-470 (2015)
[NPL 3] Endocrinology, 2021, Vol. 162, No. 12, 1-11, https://doi.org/10.1210/endocr/bqab202

### Patent Literature

[PTL 1] JP 2011-15630 A
[PTL 2] WO 2018/088240 A1 (JP 6979702 B2)

### Summary of Invention

### Technical Problem

The present invention has an object to provide a method of producing a human pluripotent stem cell-derived Leydig-like cell capable of secreting testosterone stably and persistently, and a human pluripotent stem cell-derived Leydig-like cell. The present invention has another object to provide a human pluripotent stem cell-derived Leydig-like cell that enables long-term maintenance of a Leydig-like cell secreting a sufficient amount of testosterone, and is improved in efficiency of differentiation induction.

### Solution to Problem

The inventors of the present invention have earnestly investigated in order to achieve the above-mentioned objects, and consequently have found that a Leydig-like cell capable of secreting testosterone stably and persistently can be provided by a process of producing a human pluripotent stem cell-derived Leydig-like cell, including a step of culturing a human pluripotent stem cell under a culture condition with addition of a cytokine cocktail and forcedly expressing NR5A1, in which various ways are devised for the timing of addition or removal of cAMP, suspension culture, adhesion culture, and the like. Thus, the inventors have completed the present invention.

That is, the present invention includes the following.
1. A method of producing a human pluripotent stem cell-derived Leydig-like cell, including a step of forcedly expressing NR5A1 in a human pluripotent stem cell, wherein the method includes a step of adding one or a plurality of kinds selected from the group consisting of: a WNT canonical pathway activator; bone morphogenetic protein 4 (BMP4); and vascular endothelial growth factor (VEGF) to a culture system of the human pluripotent stem cell.
2. The method of producing a human pluripotent stem cell-derived Leydig-like cell according to the above-mentioned item 1, wherein the method includes a step of forcedly expressing NR5A1 in the human pluripotent stem cell in the presence of the one or the plurality of kinds selected from the group consisting of: a WNT canonical pathway activator; BMP4; and VEGF, followed by suspension culture for from 2 days to 10 days, and then adhesion culture.
3. The method of producing a human pluripotent stem cell-derived Leydig-like cell according to the above-mentioned item 1, wherein the method includes a step of forcedly expressing NR5A1 in the human pluripotent stem cell in the presence of the one or the plurality of kinds selected from the group consisting of: a WNT canonical pathway activator; BMP4; and VEGF, followed by suspension culture until a start of formation of a germ-layer body from the human pluripotent stem cell, and then adhesion culture.
4. The method of producing a human pluripotent stem cell-derived Leydig-like cell according to the above-mentioned item 2 or 3, wherein the step of the adhesion culture includes a step of removing the WNT canonical pathway activator, the BMP4, and the VEGF that are present, followed by addition of cAMP.
5. The method of producing a human pluripotent stem cell-derived Leydig-like cell according to the above-mentioned item 4, wherein the method includes a step of removing the cAMP from the culture system in a period of from day 2 to day 40 after a start of treatment with the cAMP.
6. The method of producing a human pluripotent stem cell-derived Leydig-like cell according to the above-mentioned item 4, wherein the step of the adhesion culture includes a step of removing the WNT canonical pathway activator, the BMP4, and the VEGF that are present, followed by treatment with forskolin.
7. The method of producing a human pluripotent stem cell-derived Leydig-like cell according to the above-mentioned item 1, wherein the step of forcedly expressing NR5A1 in the human pluripotent stem cell in the presence of the one or the plurality of kinds selected from the group consisting of: a WNT canonical pathway activator; BMP4; and VEGF is performed on a culture device capable of forming one embryoid body from 50 cells to 20,000 cells.
8. The method of producing a human pluripotent stem cell-derived Leydig-like cell according to the above-mentioned item 1, wherein the step of forcedly expressing NR5A1 in the human pluripotent stem cell includes using a Tet-Off (trademark name) system that forcedly expresses NR5A1 in the absence of tetracycline or doxycycline, in a tetracycline-dependent gene expression system.
9. The method of producing a human pluripotent stem cell-derived Leydig-like cell according to the above-mentioned item 2 or 3, wherein the method includes a step of further performing suspension culture after performing the suspension culture and then the adhesion culture.
10. The method of producing a human pluripotent stem cell-derived Leydig-like cell according to the above-mentioned item 1, wherein an efficiency of differentiation induction from the human pluripotent stem cell to the human pluripotent stem cell-derived Leydig-like cell is 80% or more.
11. A human pluripotent stem cell-derived Leydig-like cell, which is produced by the method of the above-mentioned item 1.
12. The human pluripotent stem cell-derived Leydig-like cell according to the above-mentioned item 11, wherein the human pluripotent stem cell-derived Leydig-like cell is a Leydig-like cell that produces testosterone continuously for at least 60 days.
13. A human pluripotent stem cell-derived Leydig-like cell, which is obtained after the human pluripotent stem cell-derived Leydig-like cell of the above-mentioned item 11 is passaged at least once.
14. A human pluripotent stem cell-derived Leydig-like cell population, including the human pluripotent stem cell-derived Leydig-like cell of the above-mentioned item 11.
15. The human pluripotent stem cell-derived Leydig-like cell population according to the above-mentioned item 14, wherein the cell population includes 80% or more of a Leydig-like cell capable of producing testosterone.
16. The human pluripotent stem cell-derived Leydig-like cell population according to the above-mentioned item 14 or 15, wherein the cell population is subjected to immuno-isolation treatment.
17. A pharmaceutical composition for treating a disease associated with a decrease in testosterone, including the human pluripotent stem cell-derived Leydig-like cell population of the above-mentioned item 14 or 15 as an active ingredient.
18. A composition for cell transplantation, including a biocompatible membrane to which the human pluripotent stem cell-derived Leydig-like cell population of the above-mentioned item 14 or 15 adheres.
19. A device for pluripotent stem cell-derived endocrine cell transplantation, including a biocompatible membrane for transplanting a pluripotent stem cell-derived endocrine cell into a living body.
20. The device for pluripotent stem cell-derived endocrine cell transplantation according to the above-mentioned item 19, wherein the pluripotent stem cell-derived endocrine cell is a human pluripotent stem cell-derived endocrine cell capable of producing testosterone.
21. The device for pluripotent stem cell-derived endocrine cell transplantation according to the above-mentioned item 20, wherein the human pluripotent stem cell-derived endocrine cell capable of producing testosterone is the human pluripotent stem cell-derived Leydig-like cell of the above-mentioned item 11.

A. A method of transplanting a human pluripotent stem cell-derived Leydig-like cell population into a living body.
B. The transplantation method according to the above-mentioned item A, wherein the human pluripotent stem cell-derived Leydig-like cell population is transplanted into the living body together with a biocompatible membrane to which the cell population adheres.
C. The transplantation method according to the above-mentioned item A, wherein a composition for cell transplantation including a biocompatible membrane to which the human pluripotent stem cell-derived Leydig-like cell population adheres is transplanted into the living body.
D. The transplantation method according to any one of the above-mentioned items A to C, wherein the human pluripotent stem cell-derived Leydig-like cell population is a cell population including a human pluripotent stem cell-derived Leydig-like cell produced by a method including a step of adding one or a plurality of kinds selected from the group consisting of: a WNT canonical pathway activator; BMP4; and VEGF to a culture system of a human pluripotent stem cell.
E. A method of treating a disease associated with a decrease in testosterone, the method including administering a pharmaceutical composition including a human pluripotent stem cell-derived Leydig-like cell population as an active ingredient.
F. A method of treating a disease associated with a decrease in testosterone, or a method of treating a symptom requiring testosterone replacement, the method including transplanting a composition for cell transplantation including a biocompatible membrane to which a human pluripotent stem cell-derived Leydig-like cell population adheres.
G. The treatment method according to the above-mentioned item F, wherein the composition for cell transplantation is a sheet-shaped composition for cell transplantation.

### Advantageous Effects of Invention

According to the method of producing a human pluripotent stem cell-derived Leydig-like cell of the present invention, the human pluripotent stem cell-derived Leydig-like cell capable of secreting testosterone more stably and persistently can be produced. In addition, the Leydig-like cell thus produced can be transplanted into a mouse subcutaneously.

### Brief Description of Drawings

FIG. 1 is an illustration of a method of producing a Leydig-like cell of the present invention (Example 1).
FIGS. 2 show results of examining testosterone concentrations in culture supernatants in a process of producing Leydig-like cells as compared to a related-art method. FIG. 2A shows comparison between with and without adhesion culture, and FIG. 2B shows comparison between with and without a cytokine cocktail added (Experimental Example 1-1).
FIGS. 3 show results of examining effects on testosterone concentrations in culture supernatants and duration of testosterone secretion, with removal of 8-Br-cAMP from a medium on day 17 after the start of culture and with continuous presence of 1 mM 8-Br-cAMP beyond day 17 after the start of culture, in a process of producing Leydig-like cells (Experimental Example 1-2).
FIG. 4 is photographs showing cell morphologies on day 55 of culture, with removal of 8-Br-cAMP from a medium on day 17 after the start of culture and with continuous presence of 1 mM 8-Br-cAMP beyond day 17 after the start of culture, respectively, in a process of producing Leydig-like cells (Experimental Example 1-2).
FIGS. 5 are illustrations of outlines of Tet-Off^{™} system and Tet-On^{™} system for forced expression of NRSA1 gene (Experimental Example 1-3).
FIGS. 6 are photographs showing cell immunostaining images for examining expression of Leydig cell markers (17βHSD3, StAR, CYP17A1, and CYP11A1) under forced expression of NR5A1 gene using Tet-Off^{™} system and Tet-On^{™} system, in a process of producing Leydig-like cells (Experimental Example 1-3).
FIGS. 7 are photographs showing cell immunostaining images for examining expression of a Leydig cell marker (17βHSD3) under forced expression of NR5A1 gene using Tet-Off^{™} system and Tet-On^{™} system, in a process of producing Leydig-like cells (Experimental Example 1-3).
FIG. 8 shows testosterone concentrations in culture supernatants and cell morphologies after passage, for Leydig-like cells of the present invention (Experimental Example 1-4).
FIG. 9 shows testosterone concentrations in culture supernatants and cell morphologies after the second passage, for Leydig-like cells of the present invention (Experimental Example 1-4).
FIG. 10 shows testosterone concentrations in culture supernatants and cell morphologies on day 27 after freeze-thaw, for Leydig-like cells of the present invention (Experimental Example 1-5).
FIG. 11 shows testosterone concentrations and cell morphologies in immuno-isolation treatment, for Leydig-like cells of the present invention (Experimental Example 1-6).
FIG. 12 is an illustration of a method of forming a sphere (cell mass) in a method of producing Leydig-like cells of the present invention (Example 2).
FIG. 13 shows testosterone concentrations and cell morphologies in immuno-isolation treatment, for spheres (cell masses) produced from Leydig-like cells of the present invention (Example 2).
FIG. 14 is an illustration of a method of producing a composition for cell transplantation for transplanting Leydig-like cells into a mouse in a method of producing Leydig-like cells of the present invention (Example 3).
FIGS. 15 show observation of cell morphologies with culturing Leydig-like cells of the present invention on an adhesion culture dish, and with detaching the Leydig-like cells from an adhesion culture dish and culturing the cells for 2 days on a device for cell transplantation (an artificial membrane of a PET material) (Example 3).
FIG. 16A is an illustration of a method of transplanting Leydig-like cells cultured on a device for cell transplantation, together with the device for cell transplantation into a mouse, and FIG. 16B shows an observation result of transplanted cells 1 week after transplantation.
FIG. 17 shows results of harvesting a transplanted device for cell transplantation from a mouse and checking with immunostaining for a marker to be a selective indicator of a Leydig cell (LHCGR). Expression of the marker was observed at a site of presence of transplanted cells.
FIG. 18 is a diagram for illustrating a method of producing Leydig-like cells carrying introduced Venus-Akaluc gene, for examining survival of Leydig-like cells transplanted into a mouse (Example 4).
FIG. 19 is photographs showing morphologies of Leydig-like cells of the present invention on day 17 and day 19 after the start of culture (Example 4).
FIG. 20A is a diagram for illustrating a method of producing a cell sheet that includes Leydig-like cells carrying introduced Venus-Akaluc gene and a device for cell transplantation. FIG. 20B shows Leydig-like cells on day 40 after the start of culture, before the cells are seeded on a device for cell transplantation. FIG. 20C shows Leydig-like cells on day 41 after the start of culture, after the cells have been seeded on a device for cell transplantation (Example 4).
FIG. 21A is a diagram for illustrating a method of transplanting Leydig-like cells into a mouse.
FIG. 21B is photographs showing results of checking with luminescence of AkaBLI for survival of Leydig-like cells in a mouse living body on day 7 after transplantation (Example 4).

### Description of Embodiments

The present invention relates to a method of producing a human pluripotent stem cell-derived Leydig-like cell capable of secreting testosterone stably and persistently, a human pluripotent stem cell-derived Leydig-like cell produced, and a cell population of a human pluripotent stem cell-derived Leydig-like cell. A process of producing a human pluripotent stem cell-derived Leydig-like cell of the present invention includes a step of forcedly expressing NR5A1. A human pluripotent stem cell-derived Leydig-like cell capable of secreting testosterone more stably and persistently can be produced by a method including a step of subjecting a human pluripotent stem cell to forced expression of NR5A1 and then suspension culture in the presence of a cytokine cocktail required for formation of an embryoid body (EB) in a culture system, followed by adhesion culture.

As used herein, the term "Leydig-like cell" refers to a cell obtained by subjecting a pluripotent stem cell to differentiation-inducing treatment, and the cell is used differently from a naturally-occurring "Leydig cell". The "human pluripotent stem cell-derived Leydig-like cell" of the present invention refers to a cell obtained by subjecting a human pluripotent stem cell to differentiation-inducing treatment, the cell being capable of producing testosterone and/or expressing a Leydig cell marker. A Leydig cell marker is, for example, at least one marker selected from 17βHSD3, StAR, CYP17A1, and CYP11A1, and the most preferred marker is 17βHSD3. In particular, a cell found to be capable of producing testosterone is also referred to as "testosterone-producing cell" as used herein.

As used herein, the "pluripotent stem cell" only needs to be an undifferentiated cell that has "self-reproductivity", which allows proliferation while retaining an undifferentiated state, and "differentiative pluripotency", which allows differentiation into all of three germ-layer lineages. Examples thereof include an embryonic stem cell (ES cell) and an induced pluripotent stem cell (iPS cell). The method of the present invention includes using a human pluripotent stem cell. A human iPS cell closely resembles a human ES cell in properties, but has been reportedly found to differ from a murine ES cell in, for example, a network of transcription factors, epigenetics, and response to extracellular factors (STEM CELLS 2010; 28: p. 419-430; Experimental Medicine 2012; 30, No. 10: p. 1544-1548; and Journal of Clinical and Experimental Medicine 2011; 239, No. 14: p. 1247-1251). As used herein, the human pluripotent stem cell is thus considered as distinguished from a pluripotent stem cell derived from an animal except a human.

As used herein, the term "ES cell" typically refers to a pluripotent stem cell derived by transferring a cell aggregation, which is called an inner cell mass, inside a blastocyst-stage embryo, to *in vitro* culture, and isolating the cells as an undifferentiated stem cell population. Many strains of the human ES cell have already been established, and are available from ES Cell International Pte Ltd., Wisconsin Alumni Research Foundation, National Stem Cell Bank (NSCB), and the like.

As used herein, the term "iPS cell" refers to an induced pluripotent stem cell that is obtained by introducing some kinds of genes into a somatic cell and thereby inducing reprogramming of a differentiated cell without use of an ovum, an embryo, or an ES cell, and that has pluripotency and proliferative potential similar to those of an ES cell (K. Takahashi and S. Yamanaka (2006), Cell, 126: 663-676; K. Takahashi et al. (2007), Cell, 131: 861-872; J. Yu et al. (2007), Science, 318: 1917-1920; Nakagawa, M. et al., Nat. Biotechnol., 26: 101-106 (2008); and WO 2007/069666 A1). An applicable iPS cell only needs to be a human iPS cell produced by a production method known per se or a production method developed in the future. A source cell for producing an iPS cell is also not particularly limited.

A medium used for a culturing method or culture of a pluripotent stem cell, such as an ES cell or an iPS cell, is not particularly limited, and may depend on a medium or a technology known per se or developed in the future. As a medium capable of retaining undifferentiation and pluripotency of an ES cell, an iPS cell, or the like, and a medium suitable for differentiation induction thereof, there may be used, for example: commercially available basal media for mammalian cells, such as DMEM and/or DMEM/F12, supplemented with serum or a serum replacement liquid (KnockOut^{™} Serum Replacement: KSR, Thermo Fisher Scientific Inc.) and bFGF or the like; commercially available media for primate ES cells; a basal medium hESF-GRO for primate ES cell proliferation; a basal medium hESF-DIF for primate ES cell differentiation induction; and a primate ES cell proliferation medium CSTI-7. Specifically, a medium exemplified in Patent Literature 2 may be used to perform the culture.

In the process of producing a human pluripotent stem cell-derived Leydig-like cell of the present invention, NR5A1 gene is introduced into a human pluripotent stem cell and an embryoid body (EB) is formed. To form the EB, a cytokine cocktail, specifically one or a plurality of kinds of cytokines selected from the group consisting of: a WNT canonical pathway activator; bone morphogenetic protein 4 (BMP4); and vascular endothelial growth factor (VEGF) may be added to a culture system of the human pluripotent stem cell. WNT canonical pathway activation means classical Wnt pathway activation and is also referred to as activation of the β-catenin pathway where β-catenin regulates gene expression. The WNT canonical pathway activator is specifically, for example, a glycogen synthase kinase 3 (GSK-3) inhibitor, and is more specifically, for example, CHIR99021. The additive concentration of the WNT canonical pathway activator is not particularly limited, but may be appropriately selected in the range of up to 300 mM, and may be, for example, from 1 mM to 300 mM, from 1 mM to 100 mM, preferably from 10 mM to 50 mM, more preferably 20 mM. The additive concentration of BMP4 is not particularly limited, but may be appropriately selected in the range of up to 2,000 µg/mL, and may be, for example, from 10 µg/mL to 2,000 µg/mL, from 10 µg/mL to 1,000 µg/mL, preferably from 30 µg/mL to 500 µg/mL, more preferably 100 µg/mL. The additive concentration of VEGF is not particularly limited, but may be appropriately selected in the range of up to 2,000 µg/mL, and may be, for example, from 10 µg/mL to 2,000 µg/mL, from 10 µg/mL to 1,000 µg/mL, preferably from 30 µg/mL to 500 µg/mL, more preferably 100 µg/mL.

The human pluripotent stem cell is suitably subjected to suspension culture for 10 days or 8 days, more preferably 6 days after the start of culture. The EB is formed in a process of the suspension culture for from 2 days to 10 days, preferably from 6 days to 10 days, more preferably from 6 days to 8 days, most preferably 6 days after the start of culture of the human pluripotent stem cell.

A culture device for the suspension culture only needs to be a device that enables suspension culture and allows formation of an EB by three-dimensional culture or the like. In particular, a culture device that enables formation of uniformly sized EBs is suitable. Such culture device is suitably, for example, a culture device divided into microwells with a bore of from 200 µm to 20 mm, preferably from 400 µm to 1,000 µm, more preferably 400 µm or 800 µm. Specifically, AggreWell^{™}400 (StemCell Technologies Inc.) or the like may be used. The use of such culture device allows formation of uniformly sized EBs by seeding cells in a form of a single cell suspension. The culture device is suitably a culture device that enables formation of one EB from about 50 cells to about 20,000 cells, preferably from about 50 cells to about 5,000 cells, more preferably from about 100 cells to about 200 cells. For example, When an AggreWell^{™}400 6-well plate is used as the culture device, the plate includes 7,000 cavities per well and allows one EB to be formed in each of the cavities. When about 1.4×10⁶ cells are included per well, one EB is formed from 200 cells.

As used herein, the "NR5A1 gene" may be identified by, for example, GenBank Accession No. NM_004959 (SEQ ID NO: 1). A method known per se or any method developed in the future may be applied as a method of introducing the NR5A1 gene into the human pluripotent stem cell. NR5A1 cDNA may be introduced into an appropriate expression vector containing a promoter that can function in a cell to be a host by a method known per se. Specifically, a method described in Patent Literature 2 may be applied.

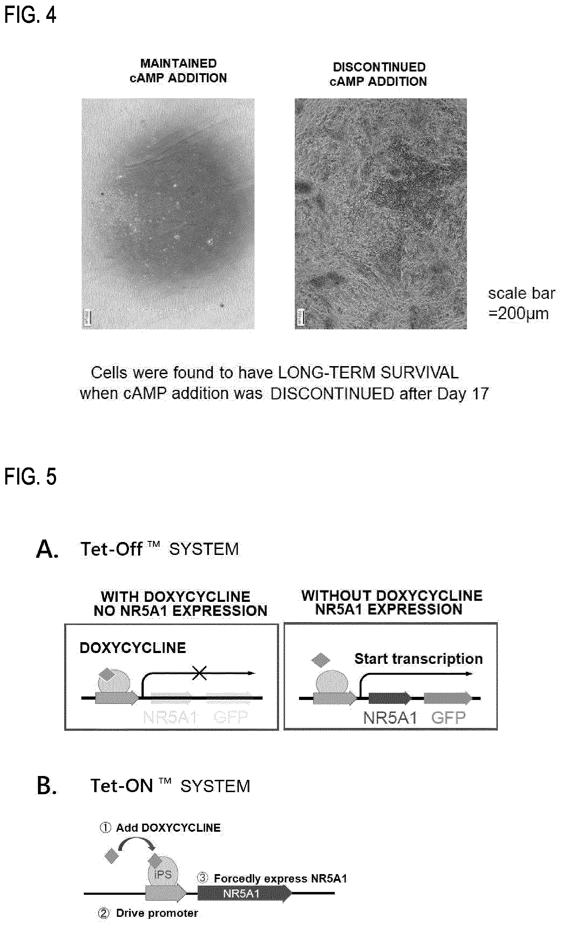

The present invention includes a step of forcedly expressing, in the human pluripotent stem cell into which the NR5A1 gene is introduced, the introduced gene. The gene may be expressed by using any gene expression system known per se or developed in the future. The timing for the gene expression may be regulated by employing a method known per se or any method developed in the future. Exemplary experimental systems capable of reversibly controlling expression of a gene of interest in a cell or the like are Tet-On^{™}/Off^{™} systems, which provide tetracycline-dependent gene expression. Such expression systems allow expression of a gene of interest to be controlled by the presence or absence of an antibiotic tetracycline or a tetracycline derivative doxycycline (Dox), which is added to a culture medium. The Tet-Off^{™} system employs, for example, the Tet repressor (TetR) and the Tet operator sequence (tetO sequence), which function in *E. coli* tetracycline-resistant operon, and allows the TetR to bind to the tetO sequence in the absence of tetracycline, leading to forced expression of NR5A1 (see FIG. 5A and FIG. 5B).

In the method of producing a human pluripotent stem cell-derived Leydig-like cell of the present invention, any of the Tet-Off^{™} system and the Tet-On^{™} system may be used to regulate expression of the NR5A1 gene. However, it is suitable to use the Tet-Off^{™} system, which provides forced expression of NR5A1 in the absence of tetracycline or doxycycline. With use of the Tet-Off^{™} system, the human pluripotent stem cell into which the NRSA1 gene has been introduced in advance is cultured in the presence of tetracycline or doxycycline, to thereby enable prevention of differentiation into a human pluripotent stem cell-derived Leydig-like cell. Then, removal of tetracycline or doxycycline at a timing required for differentiation into the Leydig-like cell allows persistent forced expression of NR5A1. Further, in the persistent forced expression of NR5A1, the removal of tetracycline or doxycycline enables avoidance of undesirable effects due to these agents and is hence suitable. Thus, the differentiation into the human pluripotent stem cell-derived Leydig-like cell can be regulated and promoted more effectively.

Tet-on/off is also described in Examples of Patent Literature 2 (WO 2018/088240 A1), but the description in Patent Literature 2 is based on the Tet-On^{™} system, which provides forced expression of NR5A1 in the presence of tetracycline or doxycycline. The Tet-off system in Examples of Patent Literature 2 is described for no forced expression of NR5A1 in the absence of tetracycline or doxycycline, and thus differs from the Tet-Off^{™} system in the present invention.

The process of producing a human pluripotent stem cell-derived Leydig-like cell of the present invention includes a step of subjecting the cell to adhesion culture after the suspension culture. The adhesion culture means that a cell is caused to adhere to a culture vessel (culture device) and cultured in a monolayer state. A petri dish (laboratory dish), a culture flask, a multiwell plate, or the like is used as the culture vessel, and there may be used a vessel having an adhesive surface coated with, for example, Matrigel^{™} (manufactured by Becton, Dickinson and Company), collagen, gelatin, laminin, heparan sulfate proteoglycan, entactin, or a combination thereof, for adhesion culture. In the step of the adhesion culture, a human mesodermal cell can be induced to differentiation into a Leydig-like cell. In the step of the adhesion culture, it is suitable to remove the cytokine cocktail (a cytokine(s) present in the culture system among one or a plurality of kinds of cytokines selected from the group consisting of: a WNT canonical pathway activator; BMP4; and VEGF) added in the culture system of the human pluripotent stem cell used in the step of the suspension culture.

In the step of the adhesion culture, it is suitable to further perform treatment with cAMP. In use as the cAMP, 8-Br-cAMP may be added at from 0.01 mM to 4 mM, preferably at from 0.1 mM to 1 mM to the medium. In addition to the treatment with the cAMP, stimulation with forskolin is preferably further performed. Forskolin may be added at from 0.1 µM to 100 µM, preferably from 2 µM to 100 µM, more preferably from 5 µM to 100 µM to the medium. The cAMP is suitably removed in a period of from day 8 to day 40 after the start of the treatment, and is removed preferably by day 30 after the start of the culture, more preferably by day 20 after start of the culture, and most preferably on day 17 after the start of the culture. Meanwhile, forskolin is preferably added in the medium even after the removal of the cAMP.

The duration of the adhesion culture is not particularly limited, and the culture may be continued as long as the culture can be performed. Further, cells may be passaged in the step of the adhesion culture. In the cell passage, it is suitable to perform treatment with a cell dissociation enzyme, such as TrypLE Select, TrypLE Express, or Accutase. Further, treatment with a ROCK inhibitor such as Y27632 may also be performed. The seeding density of the cells at the time of the passage only needs to be a concentration allowing the cells to survive and is not particularly limited, but is, for example, from 1 cell/well to 1.0×10⁶ cells/well, preferably from 1,000 cells/well to 1.0×10⁵ cells/well, more preferably about 1.0×10⁴ cells/well (9.6 cm²).

Further, the cells that have experienced the adhesion culture may be cryopreserved. A cryopreservation method, a storage condition, and the like of the cells only need to be a method performed by so-called one skilled in the art and are not particularly limited, and a method known per se or any method developed in the future may be applied. For example, a medium containing a cryoprotectant, such as dimethyl sulfoxide (DMSO) or glycerol, needs to be used as a medium for freezing, and a commercially available medium may also be used. For example, STEM-CELLBANKER^{™} (Zenogen Pharma), Recovery^{™} Cell Culture Freezing Medium (Thermo Fisher Scientific Inc.), or Synth-a-Freeze^{™} Cryopreservation Medium (Thermo Fisher Scientific Inc.) may be used as the commercially available medium. In the freezing of cells, the cells may be preserved at, for example, -80°C, or preserved at lower temperature in liquid nitrogen. A method of thawing frozen cells also only needs to be a method performed by so-called one skilled in the art and is not particularly limited, and a method known per se or any method developed in the future may be applied.

The method of producing a human pluripotent stem cell-derived Leydig-like cell of the present invention induces differentiation of the human pluripotent stem cell into the human pluripotent stem cell-derived Leydig-like cell at a high differentiation induction efficiency of 80% or more, preferably 90% or more, more preferably 95% or more. The present invention also encompasses a human pluripotent stem cell-derived Leydig-like cell and a human pluripotent stem cell-derived Leydig-like cell population produced by the method of the present invention. The human pluripotent stem cell-derived Leydig-like cell of the present invention is capable of producing testosterone continuously for at least 60 days, preferably 120 days or more. The Leydig-like cell population of the present invention is also a human pluripotent stem cell-derived Leydig-like cell population containing a Leydig-like cell capable of producing testosterone at a high ratio of 80% or more, preferably 90% or more, more preferably 95%.

The human pluripotent stem cell-derived Leydig-like cell produced by the adhesion culture in the above-mentioned step may be further subjected to suspension culture. A culture device used in the suspension culture only needs to be a culture device that enables three-dimensional culture, and is not particularly limited, but a culture device used in the step of forming the EB from the human pluripotent stem cell or the step of forcedly expressing NRSA1 in the human pluripotent stem cell may be used. Specifically, for example, a culture device divided into microwells with a bore of from 200 µm to 20 mm, preferably from 400 µm to 1,000 µm, more preferably from 400 µm or 800 µm may be used. Culture with use of such culture device enables three-dimensional culture of a human pluripotent stem cell-derived Leydig-like cell.

The present invention also encompasses a pharmaceutical composition containing a human pluripotent stem cell-derived Leydig-like cell or cell population produced by the method of the present invention as an active ingredient. A target for the pharmaceutical composition of the present invention is, for example, a disease associated with a decrease in testosterone or a symptom requiring testosterone replacement. The present invention also encompasses a therapeutic agent for a disease associated with a decrease in testosterone, containing a human pluripotent stem cell-derived Leydig-like cell or cell population produced by the method of the present invention as an active ingredient. Specific examples thereof include LOH syndrome, Klinefelter syndrome, testicular trauma, secondary hypogonadism, and a case of a biologically female human, such as a transgender, demanding male hormone replacement.

The pharmaceutical composition, or the therapeutic agent for a disease associated with a decrease in testosterone, containing a human pluripotent stem cell-derived Leydig-like cell or cell population as an active ingredient, may contain a pharmaceutically acceptable carrier in addition to the Leydig-like cell produced. The carrier is, for example, a medium in the cell culture for producing the Leydig-like cell. In addition, the human pluripotent stem cell-derived Leydig-like cell may be subjected to immuno-isolation treatment.

The present invention also encompasses a device for human pluripotent stem cell-derived endocrine cell transplantation. In other words, the present invention also encompasses a device before adhesion culture of the human pluripotent stem cell-derived Leydig-like cell population of the present invention. As used herein, the term "device for pluripotent stem cell-derived endocrine cell transplantation" refers to a device capable of transplanting a pluripotent stem cell-derived endocrine cell, the device allowing, in adhesive culture of a pluripotent stem cell-derived endocrine cell on the device, effective secretion of a substance produced by the pluripotent stem cell-derived endocrine cell, such as a hormone, specifically testosterone. A material that may be used for the device for pluripotent stem cell-derived endocrine cell transplantation needs to have biocompatibility as a property of providing no harmful effect on a living body. A biocompatible material suitably has affinity with a living body, and may be a material biologically absorbed after the human pluripotent stem cell-derived Leydig-like cell population transplanted establishes engraftment to a living body. Examples of such material include synthetic polymers, such as polyethylene terephthalate (PET), poly-L-lactic acid (PLLA), polyglycolic acid (PGA), a copolymer of lactic acid and glycolic acid (PLGA), and polycaprolactone (PCL). Natural polymers, such as collagen, gelatin, glycosaminoglycan, chitin, chitosan, hyaluronic acid, and polypeptide, may be used. The shape of the device for pluripotent stem cell-derived endocrine cell transplantation is not particularly limited, but, for example, a sheet-shaped one such as a biocompatible membrane may be used.

The present invention also encompasses a composition for cell transplantation including a device for pluripotent stem cell-derived endocrine cell transplantation to which the human pluripotent stem cell-derived Leydig-like cell population of the present invention adheres. The human pluripotent stem cell-derived Leydig-like cell adhering to the device for cell transplantation may be further subjected to immuno-isolation treatment. The composition for cell transplantation may be produced by adhesion culture of the human pluripotent stem cell-derived Leydig-like cell population of the present invention on the device for cell transplantation. The shape of the composition for cell transplantation only needs to be a shape allowing transplantation of a cell and is not particularly limited, but may be, for example, a sheet shape or a capsule shape.

As used herein, the term "immuno-isolation treatment" refers to treatment so as to allow water, nutrients, or hormones to pass through, but prohibit immune cells and transplanted cells to pass through, and inhibit rejection of cells transplanted under a local environment, without hampering angiogenesis. A method known per se or any method developed in the future may be applied as the immuno-isolation treatment. For example, the immuno-isolation treatment may be performed by embedding cells in, for example, beads or capsules of alginate gel, agarose, an anisotropic material, polysulfone (PSF), a nanofiber mat, polyimide, tetrafluoroethylene/polytetrafluoroethylene (PTFE), ePTFE, polyacrylonitrile, polyethersulfone, an acrylic resin, cellulose acetate, cellulose nitrate, polyamide, or a hydroxypropyl methylcellulose (HPMC) membrane. Such immuno-isolation treatment allows avoiding problems, such as an immunorejection response associated with cell transplantation, or diffusion or invasion of cells. The immuno-isolation treatment enables the human pluripotent stem cell-derived Leydig-like cell produced, even not from one's own cell (not from an autologous cell), to be used for treating a pathology requiring continuous supply of a substance produced by the human pluripotent stem cell-derived Leydig-like cell, specifically testosterone, to an organism.

The present invention also encompasses a method of transplanting a human pluripotent stem cell-derived Leydig-like cell population produced by the method of the present invention. Transplantation may be achieved by a method of transplanting the cell population together with a device for pluripotent stem cell-derived endocrine cell transplantation to which the cell population adheres, into a desired site in a living body, preferably subcutaneously. A target of the transplantation of the human pluripotent stem cell-derived Leydig-like cell population of the present invention is, for example, a disease associated with a decrease in testosterone or a symptom requiring testosterone replacement, as is the case with the target of the pharmaceutical composition described above. Specific examples thereof include LOH syndrome, Klinefelter syndrome, testicular trauma, secondary hypogonadism, and a case of a biologically female human, such as a transgender, demanding male hormone replacement.

The present invention also encompasses a method of treating a disease associated with a decrease in testosterone, including administering a pharmaceutical composition containing a human pluripotent stem cell-derived Leydig-like cell population as an active ingredient, a method of treating a disease associated with a decrease in testosterone, including transplanting a composition for cell transplantation including a device for pluripotent stem cell-derived endocrine cell transplantation to which a human pluripotent stem cell-derived Leydig-like cell population adheres, or a treatment method for a symptom requiring testosterone replacement.

### Examples

The present invention is described in detail below with reference to Examples for further understanding of the present invention, but it should be appreciated that the present invention is not limited to these Examples.

### (Example 1) Method of producing Leydig-like Cells

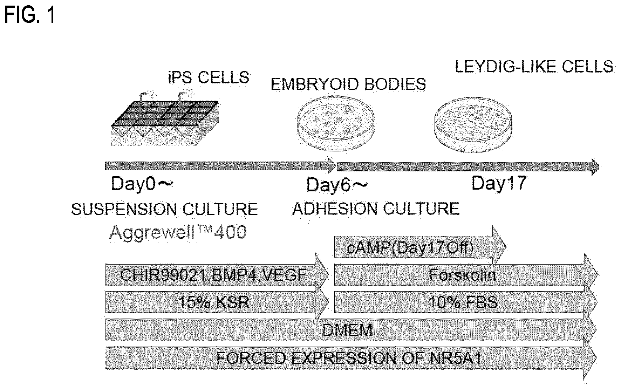

In this Example, a method of producing Leydig-like cells is described (see FIG. 1).

### 1. Cells for Use

In this Example, iPS cells (FFPB3AB4) was used to perform differentiation-inducing treatment.

### 2. NR5A1 Gene

According to the method described in Example 1 of Patent Literature 2, the full-length NR5A1 gene (cDNA) identified as GenBank Accession No. NM_004959 (SEQ ID NO: 1) was cloned, and a NRSA1 gene expression vector was constructed and transfected in iPS cells (FFPB3AB4). NR5A1 was forcedly expressed by Tet-Off^{™} Advanced Inducible Expression System (Clontech).

### 3. Differentiation Induction into Leydig-like Cells

The human iPS cells having the NR5A1 gene forcedly expressed by the Tet-Off^{™} system (KW107_121-3_NR5A1_Leydig_P12+2(EX564)) were seeded at 1.4×10⁶ cells per well in an AggreWell^{™}400 6-well plate. In culture, a differentiation induction medium (DMEM medium (Invitrogen) containing 15% KSR (Thermo Fisher Scientific Inc.)) containing 20 mM CHIR99021, 100 µg/mL BMP4, and 100 µg/mL VEGF as a cytokine cocktail was used to perform culture for 6 days. The AggreWell^{™}400 6-well plate has 7,000 cavities per well, and one EB is formed from about 200 cells in one cavity after the culture for 6 days.

KW107_121-3_NR5A1_Leydig_P12+2(EX564) forming EBs was further cultured for 11 days in a plate for adhesion culture (Nunc^{™} Cell-Culture Treated Multidishes) through use of 10% FBS-containing DMEM medium (Invitrogen) containing 1 mM 8-Br-cAMP, 10 µM Y27632, 100 µM forskolin, and no KSR, followed by removal of 8-Br-cAMP on day 17. Subsequently, the cells were maintained in adhesion culture in 10% FBS-containing DMEM medium (Invitrogen) containing 100 µM forskolin.

In each of Experimental Examples below, KW107_121-3_NR5A1_Leydig_P12+2(EX564) or cells treated with a Tet-On^{™} system (KW111_3AB4_2S6_NR5A1_Leydig) were variously investigated for differentiation-inducing treatment conditions, and Leydig-like cells thus produced were examined for their properties.

### (Experimental Example 1-1) Effects of Adhesion Culture and Addition of Cytokine Cocktail on Amount of Testosterone Secretion

In this Example, cells produced according to the production method in Example 1 (Tet-On^{™} system) and cells produced by a related-art method (see Example 1 of Patent Literature 2) (both are KW111_3AB4_2S6_NR5A1_hiPSC-derived Leydig-like cells) were measured for the amounts of testosterone in culture supernatants with and without adhesion culture and with and without addition of a cytokine cocktail.

### 1. With or Without Adhesion Culture

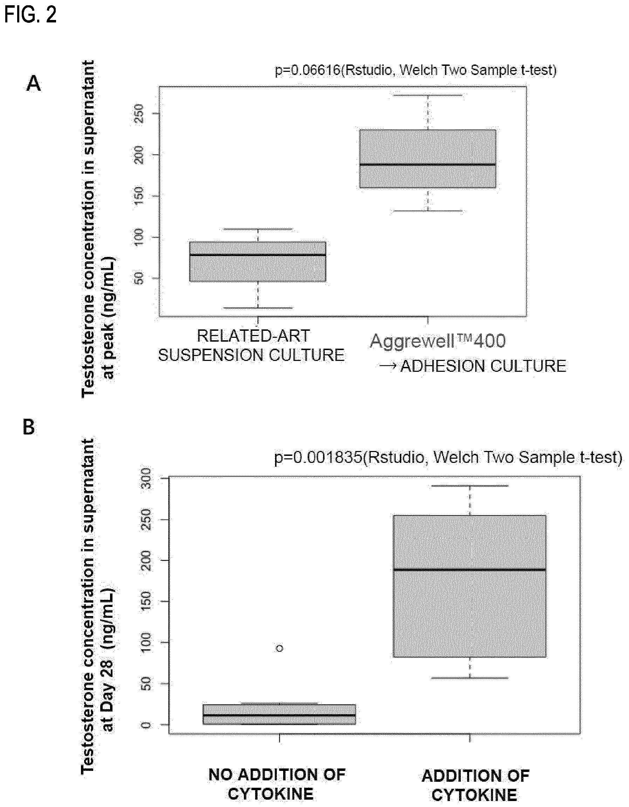

FIG. 2A shows testosterone concentrations at peak (on from day 16 to day 26 of culture) in culture supernatants of cells that experienced suspension culture in an AggreWell^{™}400 6-well plate and then adhesion culture, and of cells derived from a related-art method (in continuing suspension culture with three-dimensional culture plates (PrimeSurface^{™}: MS-9096M and MS-90240)) as Comparative Example. The testosterone concentration was measured by electro chemiluminescence immunoassay (ECLIA). The results revealed that the system in which suspension culture was performed in AggreWell^{™}400 and was then switched to adhesion culture provided a significantly higher testosterone concentration.

### 2. With or without Addition of Cytokine

FIG. 2B shows testosterone concentrations in culture supernatants on day 28 of culture, in a cell system with addition of a cytokine cocktail by day 6 of culture in continuing suspension culture with three-dimensional culture plates (PrimeSurface^{™}: MS-9096M and MS-90240) and in a cell system of continuous suspension culture without addition of a cytokine cocktail by day 6. The results revealed that the system with addition of a cytokine cocktail had a significantly higher testosterone concentration and a larger amount of testosterone secretion.

### (Experimental Example 1-2) Effect of Addition of 8-Br-cAMP on Amount of Testosterone Secretion

In this Experimental Example, an effect of 8-Br-cAMP in a process of producing Leydig-like cells was investigated. Testosterone concentrations in culture supernatants were examined for: a case of adding 1 mM 8-Br-cAMP with the start of adhesion culture on day 6 after the start of culture, and removing 8-Br-cAMP from a medium on day 17 after the start of culture; and a case in which the medium continuously contained 1 mM 8-Br-cAMP beyond day 17 after the start of culture. A method of measuring the testosterone concentration was performed by the same technique as in Experimental Example 1.

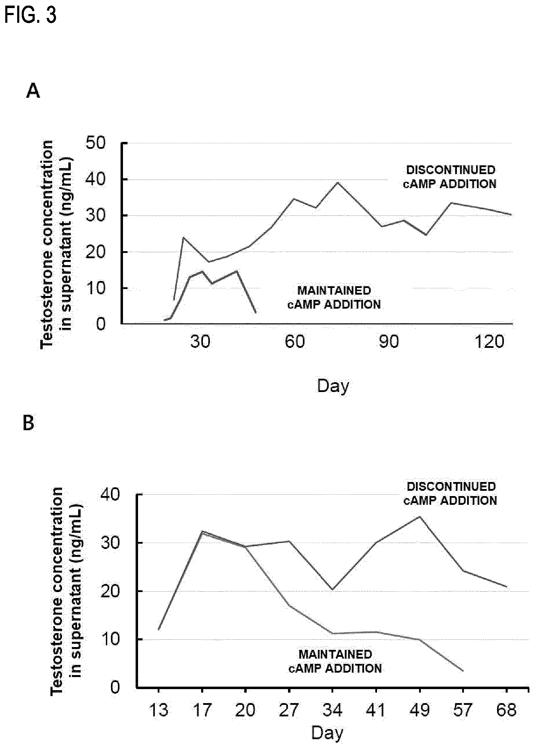

The results revealed that the removal of 8-Br-cAMP in the middle of the culture was better for maintaining testosterone secretion (FIGS. 3). The results also revealed that the removal of 8-Br-cAMP in the middle of the culture was better for long-term survival of cells (FIG. 4).

### (Experimental Example 1-3) Expression of Leydig Cell Marker

In this Experimental Example, human iPS cells having the NR5A1 gene forcedly expressed with a Tet-Off^{™} system or a Tet-On^{™} system were examined for expression of Leydig cell markers 17βHSD3, StAR, CYP17A1, and CYP11A1 by cell immunostaining. Tet-Off^{™} Advanced Inducible Expression System (Clontech) or Tet-On^{™} Advanced Inducible Expression System (Clontech) was used as the Tet-Off^{™} system or the Tet-On^{™} system (see FIGS. 5).

### -Cells treated with Tet-Off^{™} system: KW107_121-3_NR5A1_Leydig_P12+2(EX564)

### ·Cells treated with Tet-On^{™} system: KW111_3AB4_2S6_NR5A1_Leydig

In the Tet-Off^{™} system, the Leydig-like cells produced by the method of Example 1 were passaged four times, and cells on day 4 after the fourth passage were measured. In the Tet-On^{™} system, differentiation induction from iPS cells was started, and cells on day 25 were measured.

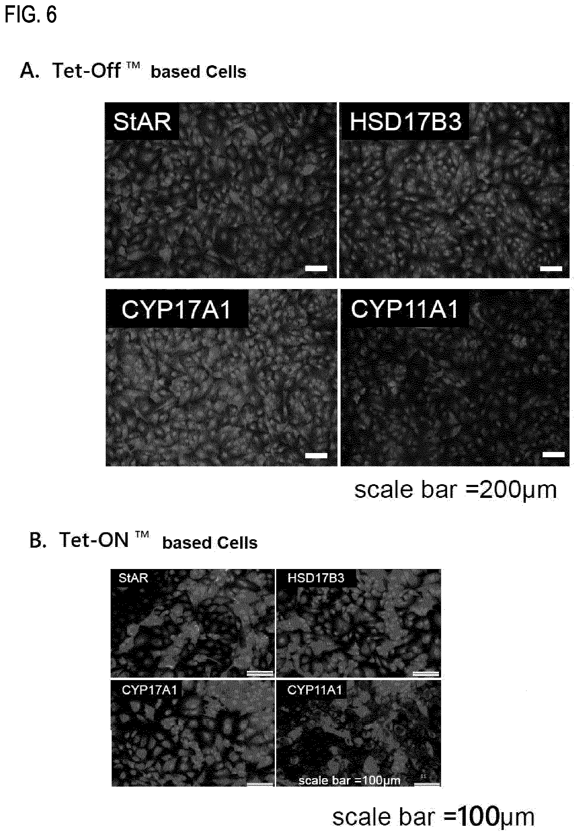

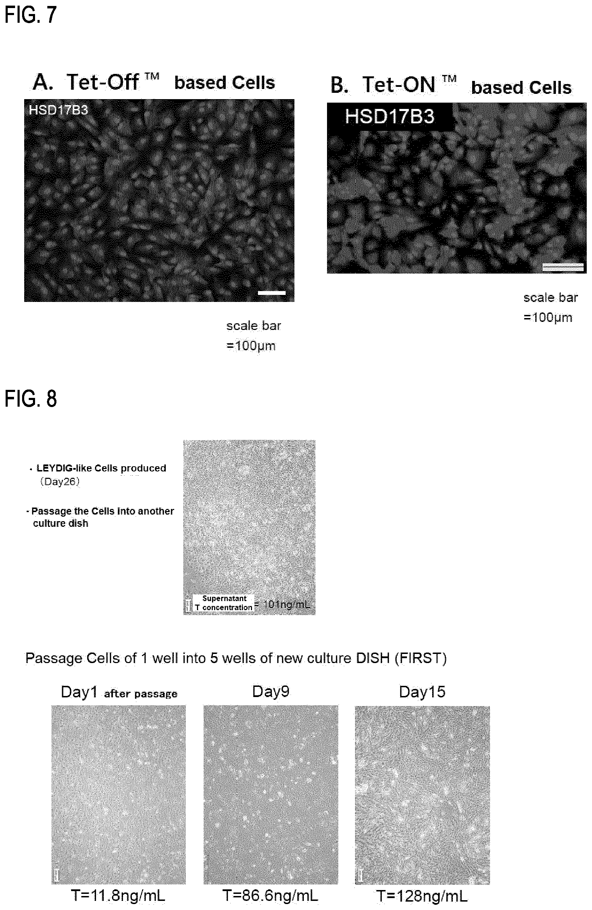

The results revealed that the Leydig cell markers were expressed even when the NR5A1 gene was forcedly expressed with any of the Tet-Off^{™} system and the Tet-On^{™} system, but that the cells having the NRSA1 gene forcedly expressed with the Tet-Off^{™} system expressed the Leydig cell markers 17βHSD3, StAR, and CYP17A1 with higher densities (FIGS. 6). In particular, 99.9% or more of all the cells were 17βHSD3-positive (FIGS. 7). This revealed that long-term forced expression of the NR5A1 gene by the Tet-Off^{™} system exhibited extremely high efficiency of differentiation induction into Leydig-like cells and enabled acquiring a Leydig-like cell population having a higher purity.

(Experimental Example 1-4) Amount of Testosterone Secretion in Cells after Passage In this Experimental Example, cells after passage were measured for testosterone concentrations in culture supernatants. Measurement of the testosterone concentrations was performed by the same technique as in Experimental Example 1.

Cells cultured by the method of Example 1 (Tet-Off^{™} system) (day 26 after the start of culture) were treated with a cell dissociation enzyme (0.5× TrypLE Select), harvested, and suspended in a maintenance medium (10% FBS+100 µM forskolin-containing DMEM medium (Invitrogen)) to provide a cell suspension, followed by passage from 1 well into 5 wells. The seeding density of the cells at this time was about 1×10⁴ cells/well (9.6 cm²). The cells on day 1, day 9, and day 15 after passage were measured for testosterone concentrations in culture supernatants. Testosterone secretion was identified as 11.8 ng/mL on day 1, 86.6 ng/mL on day 9, and 128 ng/mL on day 15 after passage (FIG. 8).

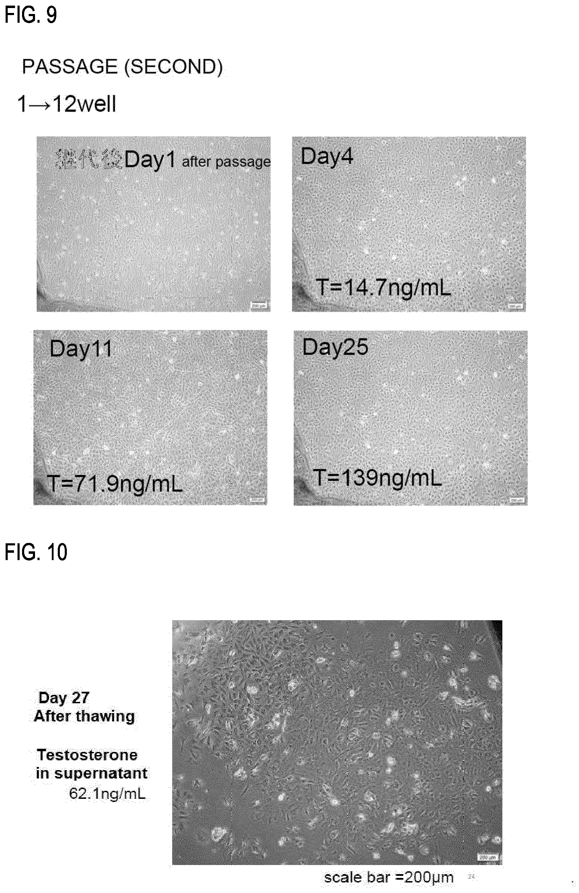

As in the first passage, cells subjected to adhesion culture for 18 days after the first passage were treated with a cell dissociation enzyme (0.5× TrypLE Select), and suspended in a maintenance medium (10% FBS+100 µM forskolin-containing DMEM medium (Invitrogen)) to provide a cell suspension, followed by passage from 1 well into 12 wells. The seeding density of the cells at this time was about 1×10⁴ cells/well (9.6 cm²). The cells on day 1, day 9, and day 15 after passage were measured for testosterone concentrations in culture supernatants. Testosterone secretion was identified as 14.7 ng/mL on day 4, 71.9 ng/mL on day 11, and 139 ng/mL on day 25 after passage (FIG. 9).

### (Experimental Example 1-5) Amount of Testosterone Secretion in Cells after Freezing and Thawing

In this Experimental Example, cells atter freezing and thawing were measured for a testosterone concentration in a culture supernatant. Measurement of the testosterone concentration was performed by the same technique as in Experimental Example 1.

Cells cultured by the method of Example 1 (Tet-Off^{™} system) (day 48 after the start of culture) were treated with a cell dissociation enzyme (0.5× TrypLE Select), suspended in a medium for freezing (STEM-CELLBANKER), dispensed into cryopreservation vials, and cryopreserved in liquid nitrogen. The cryopreserved cells were removed from liquid nitrogen storage, thawed, and suspended and cultured in a maintenance medium (10% FBS+100 µM forskolin-containing DMEM medium, Invitrogen) according to a common method.

The cells on day 27 after freezing and thawing were measured for a testosterone concentration in a culture supernatant. The result revealed testosterone secretion at 62.1 ng/mL (FIG. 10).

### (Experimental Example 1-6) Amount of Testosterone Secretion in Leydig-like Cells subjected to Immuno-isolation Treatment

Leydig-like cells produced by the method of Example 1 (Tet-Off^{™} system) were passaged once, and the cells on day 18 after the passage were subjected to immuno-isolation treatment according to the following method. A culture supernatant containing the Leydig-like cells subjected to the immuno-isolation treatment was measured for a testosterone concentration. Measurement of the testosterone concentration was performed by the same technique as in Experimental Example 1.
(1) Apply 2 mL/well of a calcium chloride solution into a Nunc^{™} Cell-Culture Treated Multidishes 12-well plate.
(2) Remove a maintenance medium of KW107_121-3_NR5A1_Leydig_P12+2(EX564) in adhesion culture on day 18 after passage.
(3) Apply 400 µL/well of 0.5× TrypLE select.
(4) Place the cells at 37°C in a 5% CO₂ incubator, and leave them to stand for 2 minutes.
(5) Retrieve the cells from the incubator, and remove 0.5× TrypLE select.
(6) Wash the cells with 1×PBS.
(7) Apply 300 µL/well of a sodium alginate solution.
(8) Suspend the cells by pipetting.
(9) Pour 200 µL of the suspension of the item (8) into a well of the item (1) with a pipette chip.
(10) Leave the suspension at normal temperature for 5 minutes to provide Leydig-like cells embedded in alginate beads.
(11) Remove a solution except the beads.
(12) Add 2 mL/well of a maintenance medium (10% FBS+100 µM forskolin-containing DMEM medium).

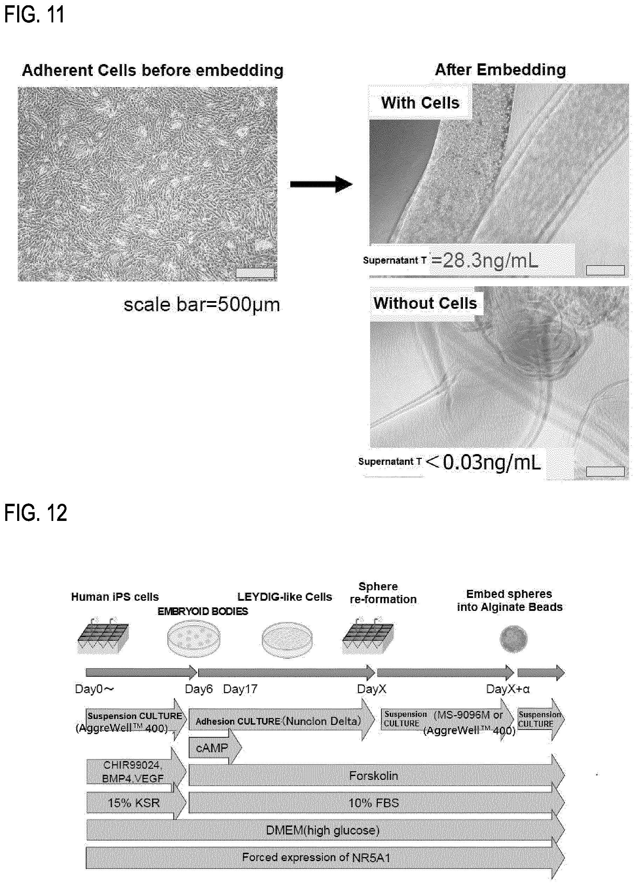

The result revealed testosterone secretion at 28.3 ng/mL from the Leydig-like cells subjected to the immuno-isolation treatment (FIG. 11).

### (Example 2) Production of Leydig-like Cells 2

iPS cells (FFPB3AB4) were used and cultured by the same techniques as shown in the sections 1 to 3 of Example 1, and after removal of 8-Br-cAMP on day 17 of culture, the cells were further subjected to maintenance culture in 10% FBS-containing DMEM medium (Invitrogen) containing 100 µM forskolin. The cells were treated with a cell dissociation enzyme (0.5× TrypLE Select) and harvested on day 30 after the start of culture. The cells were washed with a medium by a common method, then suspended in a maintenance medium (10% FBS+100 µM forskolin-containing DMEM medium) to provide a cell suspension, and seeded at 2.0×10⁵ cells per well in an AggreWell^{™}400 6-well plate to form small globular cell masses (small spheres) of the cells. As Comparative Example, cells were seeded at 1.25×10⁴ cells per well in a three-dimensional culture plate (PrimeSurface^{™}: MS-9096M) to form large globular cell masses (large spheres) of the cells (FIG. 12).

The large spheres and the small spheres formed over 4 days were subjected to immuno-isolation treatment by the following method, and then cultured for 3 days, and the culture supernatants containing the Leydig-like cells thus obtained were measured for testosterone concentrations. Measurement of the testosterone concentrations was performed by the same technique as in Experimental Example 1.
(1) Apply 2 mL/well of a calcium chloride solution into a Nunc^{™} Cell-Culture Treated Multidishes 12-well plate.
(2) Collect each of large spheres and small spheres from a corresponding suspension culture into a single tube, and remove a maintenance medium.
(3) Dispense 300 µL/well of a sodium alginate solution.
(4) Pour 1,000 µL of each of the suspensions of the item (3) into an independent well of the item (1) with a pipette chip.
(5) Leave the suspension at normal temperature for 5 minutes to provide Leydig-like cells embedded in alginate beads.
(6) Remove a solution except spheres embedded in the beads.
(7) Add 2 mL/well of a maintenance medium (10% FBS+100 µM forskolin-containing DMEM medium), followed by culture.

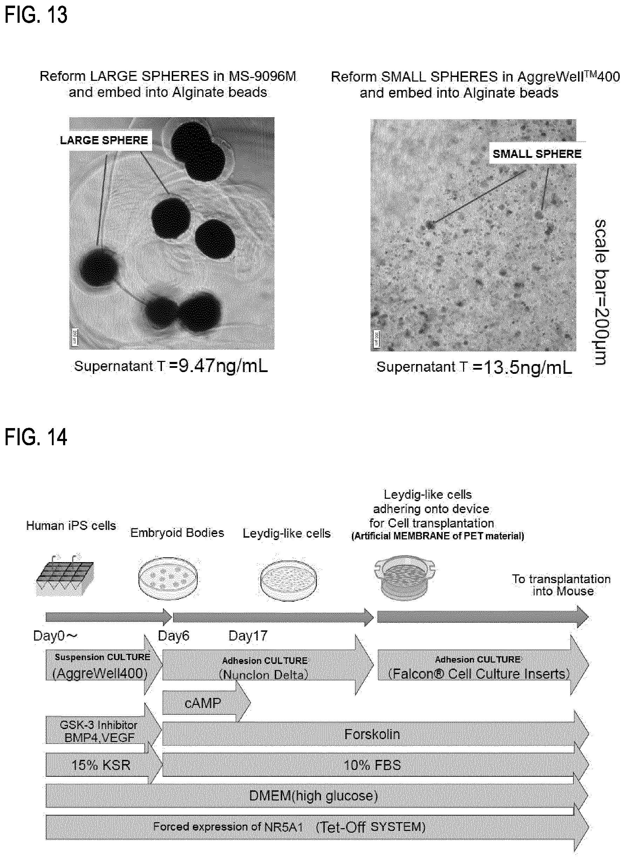

The results revealed that the Leydig-like cells subjected to the immuno-isolation treatment for the large spheres provided testosterone secretion at 9.47 ng/mL, while the Leydig-like cells subjected to the immuno-isolation treatment for the small spheres provided testosterone secretion at 13.5 ng/mL (FIG. 13). The foregoing revealed that formation of the small spheres provided higher testosterone secretion.

### (Example 3) Transplantation of Leydig-like Cells

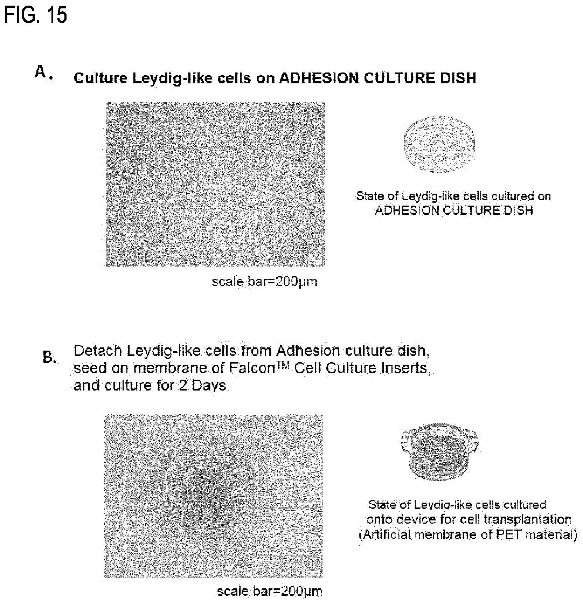

This Example examined whether the Leydig-like cells of the present invention were able to be transplanted into a mouse, through use of female mice. iPS cells (FFPB3AB4) were used and cultured by the same techniques as shown in the sections 1 to 3 of Example 1, and after removal of 8-Br-cAMP on day 17 of culture, the cells were further subjected to maintenance culture in 10% FB S-containing DMEM medium (Invitrogen) containing 100 µM forskolin. The cells were treated with a cell dissociation enzyme (0.5× TrypLE Select) on day 20 after the start of culture, and harvested. The cells were washed with a medium by a common method, and then suspended in a maintenance medium (10% FBS+100 µM forskolin-containing DMEM medium) to provide a cell suspension. Falcon^{™} Cell Culture Inserts was prepared as a device for cell transplantation including an artificial membrane of PET material, and 4.5×10⁵ cells were seeded in the device for cell transplantation and cultured for 2 days to provide a cell sheet (FIG. 14). Morphologies of the Leydig-like cells adhering onto adhesion culture dishes and devices for cell transplantation were observed (FIGS. 15).

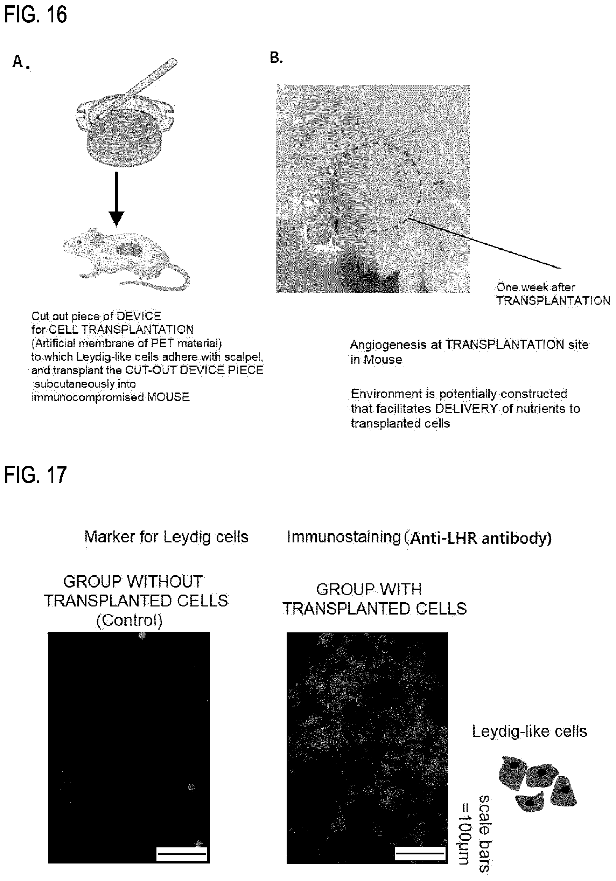

The cell sheet including the device for cell transplantation was transplanted subcutaneously into an immunocompromised mouse (24-week-old, female) (FIG. 16A). One week after the transplantation, the device for cell transplantation was harvested from the mouse, and the transplanted Leydig-like cells were observed. Angiogenesis in the mouse was observed at a site of the cell sheet transplanted, suggesting that an environment that facilitated delivery of nutrients to the transplanted Leydig-like cells was potentially established (FIG. 16B). Whether the transplanted Leydig-like cells expressed a Leydig cell marker was examined by immunostaining with an antibody against an indicator marker (LHCGR) (anti-LHR antibody). At a site where the transplanted cells were present, expression of the marker was observed (FIG. 17).

The foregoing revealed that the Leydig-like cells of the present invention were able to be transplanted into the mouse, and were present without peeling off from the device for cell transplantation.

### (Example 4) Examination of Survival of Transplanted Leydig-like Cells

This Example examined whether the Leydig-like cells of the present invention transplanted into a mouse survived in the body of the mouse, through use of an artificial bioluminescence system AkaBLI (RIKEN).

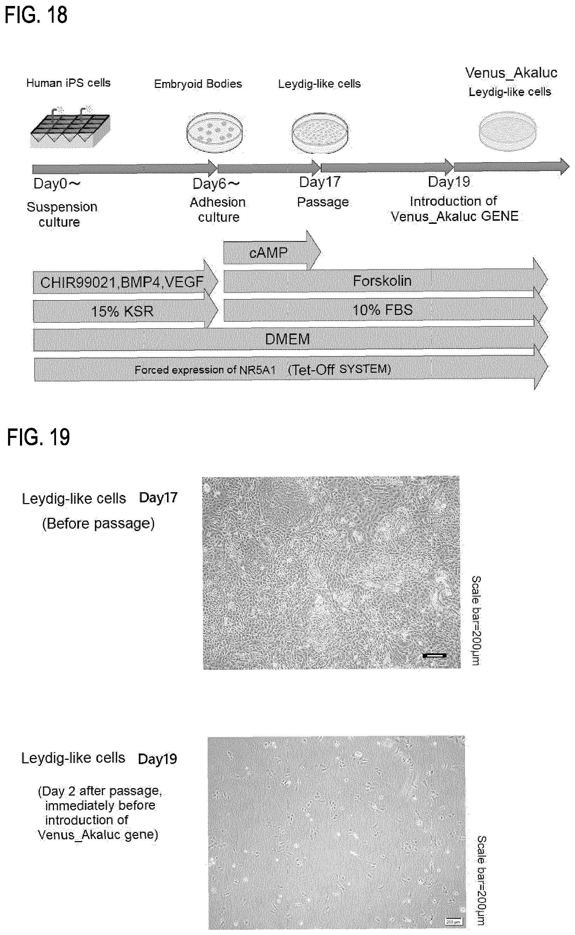

iPS cells (FFPB3AB4) were used and cultured by the same techniques as shown in the sections 1 to 3 of Example 1, and on day 17, treated with a cell dissociation enzyme (0.5× TrypLE Select), harvested, and passaged in 10% FBS-containing DMEM medium (Invitrogen) containing 100 µM forskolin and no 8-Br-cAMP. On day 2 after passage (day 19 after the start of culture), a retrovirus vector was used to introduce a Venus-Akaluc gene (RIKEN) (FIG. 18). Akaluc is an artificial enzyme that generates luminescence from AkaBLI when combined with AkaLumine, a substrate. FIG. 19 shows Leydig-like cells on day 17 after the start of culture and cells immediately before introduction of the Venus-Akaluc gene (on day 19 after the start of culture).

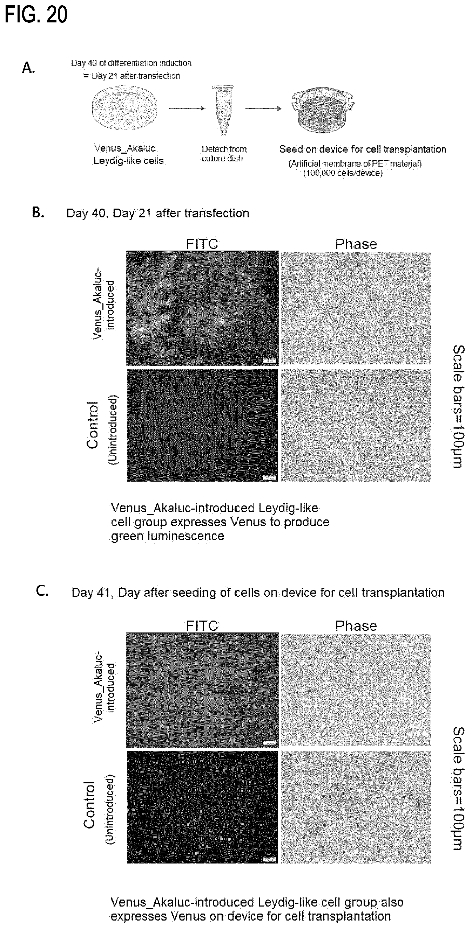

On day 21 after introduction of the Venus-Akaluc gene (day 40 after the start of culture), cells were treated with a cell dissociation enzyme (0.5× TrypLE Select) and harvested. The cells were washed with a medium by a common method, and then suspended in a maintenance medium (10% FB S+100 µM forskolin-containing DMEM medium) to provide a cell suspension. The cell suspension was seeded at 1.0×10⁵ cells into a device for cell transplantation including an artificial membrane of a PET material as in Example 3 to produce a cell sheet (FIG. 20A). FIG. 20B shows cells on day 21 after introduction of the Venus-Akaluc gene, and FIG. 20C shows cells on the day after seeding in the device for cell transplantation. In each case, cells untransfected with the Venus-Akaluc gene were used as a control. The Venus-Akaluc geneintroduced cells expressed Venus, and even after seeding in the device for cell transplantation, expression of Venus was detected on the device (FIG. 20B and FIG. 20C).

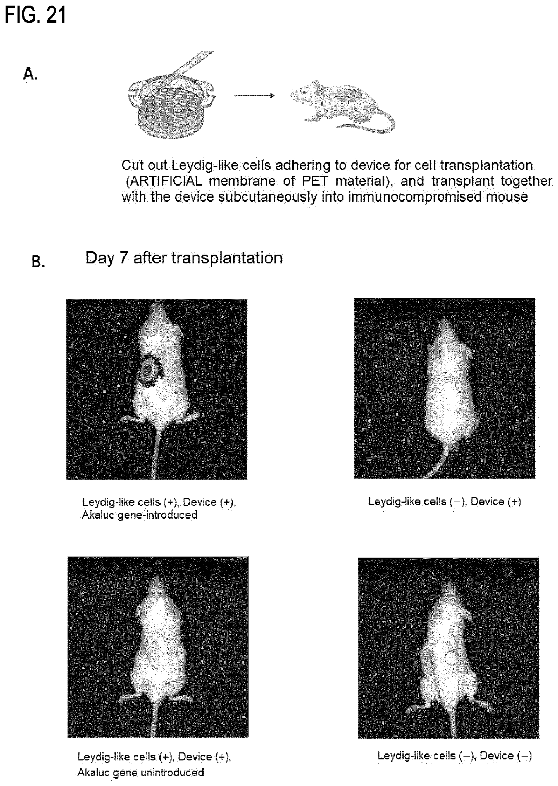

The cell sheet including the device for cell transplantation was transplanted subcutaneously into a immunocompromised mouse (11-week-old, female) (FIG. 21A). Seven days after the transplantation, AkaLumine hydrochloride was injected intraperitoneally, followed by observation of *in vivo* luminescence of AkaBLI with IVIS Imaging System (PerkinElmer) to examine expression of Venus. Luminescence by AkaBLI was identified in the cell sheet having the Leydig-like cells having the Akaluc gene introduced, proving that the transplanted Leydig-like cells survived in the living body of the mouse (FIG. 21B).

### Industrial Applicability

As described in detail above, the Leydig-like cell capable of secreting testosterone stably and persistently can be provided by a process of producing a human pluripotent stem cell-derived Leydig-like cell, including a step of culturing a human pluripotent stem cell under a culture condition with addition of a cytokine cocktail to forcedly express NR5A1, in which various ways are devised for the timing of addition or removal of cAMP, suspension culture, adhesion culture, and the like. The human pluripotent stem cell-derived Leydig-like cell produced by the method of the present invention is capable of producing testosterone stably over a long period, and expresses any one of 17βHSD3, StAR, CYP17A1, and CYP11A1.

Further, in the step of forcedly expressing NR5A1, NR5A1 can be continuously forcedly expressed in the absence of tetracycline by using the Tet-Off^{™} system, which employs the Tet repressor (TetR) and the Tet operator sequence (tetO sequence), which function in *E. coli* tetracycline-resistant operon, and allows the TetR to bind to the tetO sequence in the absence of tetracycline. This enables production of Leydig-like cells at a desired timing by preserving human pluripotent stem cell-derived Leydig-like cells before differentiation-inducing treatment in the presence of tetracycline, doxycycline or the like, and removing tetracycline, doxycycline or the like when differentiation induction is required. In addition, Leydig-like cells can be produced in the absence of tetracycline or doxycycline, and hence undesirable effects due to these agents can be avoided.

The Leydig-like cell produced by the method of the present invention can be passaged and cryopreserved. In addition, the Leydig-like cell of the present invention can be transplanted together with the composition for cell transplantation of the present invention, that is, the device for human pluripotent stem cell-derived endocrine cell transplantation, such as a biocompatible membrane, to which the Leydig-like cell of the present invention adheres, into a desired site in a living body, preferably subcutaneously. Further, the immuno-isolation treatment also provides a function as a testosterone-producing cell. The human pluripotent stem cell-derived Leydig-like cell of the present invention may be applied to a person who has a disease associated with a decrease in testosterone or a symptom requiring testosterone replacement. Specific examples thereof include LOH syndrome, Klinefelter syndrome, testicular trauma, secondary hypogonadism, and a case of a biologically female human, such as a transgender, demanding male hormone replacement. The human pluripotent stem cell-derived Leydig-like cell used for those cases is useful because transplantation of the cell subjected to the immuno-isolation treatment allows water, nutrients, or hormones to pass through, but prohibits immune cells, transplanted cells, and the like to pass through, and thus enables preventing a rejection response associated with cell transplantation, and diffusion and invasion of cells.

## Claims

1. A method of producing a human pluripotent stem cell-derived Leydig-like cell, comprising a step of forcedly expressing NR5A1 in a human pluripotent stem cell,
wherein the method comprises a step of adding one or a plurality of kinds selected from the group consisting of: a WNT canonical pathway activator; bone morphogenetic protein 4 (BMP4); and vascular endothelial growth factor (VEGF) to a culture system of the human pluripotent stem cell.

2. The method of producing a human pluripotent stem cell-derived Leydig-like cell according to claim 1, wherein the method comprises a step of forcedly expressing NR5A1 in the human pluripotent stem cell in the presence of the one or the plurality of kinds selected from the group consisting of: a WNT canonical pathway activator; BMP4; and VEGF, followed by suspension culture for from 2 days to 10 days, and then adhesion culture.

3. The method of producing a human pluripotent stem cell-derived Leydig-like cell according to claim 1, wherein the method comprises a step of forcedly expressing NR5A1 in the human pluripotent stem cell in the presence of the one or the plurality of kinds selected from the group consisting of: a WNT canonical pathway activator; BMP4; and VEGF, followed by suspension culture until a start of formation of a germ-layer body from the human pluripotent stem cell, and then adhesion culture.

4. The method of producing a human pluripotent stem cell-derived Leydig-like cell according to claim 2 or 3, wherein the step of the adhesion culture comprises a step of removing the WNT canonical pathway activator, the BMP4, and the VEGF that are present, followed by addition of cAMP.

5. The method of producing a human pluripotent stem cell-derived Leydig-like cell according to claim 4, wherein the method comprises a step of removing the cAMP from the culture system in a period of from day 2 to day 40 after a start of treatment with the cAMP.

6. The method of producing a human pluripotent stem cell-derived Leydig-like cell according to claim 4, wherein the step of the adhesion culture comprises a step of removing the WNT canonical pathway activator, the BMP4, and the VEGF that are present, followed by treatment with forskolin.

7. The method of producing a human pluripotent stem cell-derived Leydig-like cell according to claim 1, wherein the step of forcedly expressing NR5A1 in a human pluripotent stem cell in the presence of one or a plurality of kinds selected from the group consisting of: a WNT canonical pathway activator; BMP4; and VEGF is performed on a culture device capable of forming one embryoid body from 50 cells to 20,000 cells.

8. The method of producing a human pluripotent stem cell-derived Leydig-like cell according to claim 1, wherein the step of forcedly expressing NR5A1 in a human pluripotent stem cell comprises using a Tet-Off (trademark name) system that forcedly expresses NR5A1 in the absence of tetracycline or doxycycline, in a tetracycline-dependent gene expression system.

9. The method of producing a human pluripotent stem cell-derived Leydig-like cell according to claim 2 or 3, wherein the method comprises a step of further performing suspension culture after performing the suspension culture and then the adhesion culture.

10. The method of producing a human pluripotent stem cell-derived Leydig-like cell according to claim 1, wherein an efficiency of differentiation induction from the human pluripotent stem cell to the human pluripotent stem cell-derived Leydig-like cell is 80% or more.

11. A human pluripotent stem cell-derived Leydig-like cell, which is produced by the method of claim 1.

12. The human pluripotent stem cell-derived Leydig-like cell according to claim 11, wherein the human pluripotent stem cell-derived Leydig-like cell is a Leydig-like cell that produces testosterone continuously for at least 60 days.

13. A human pluripotent stem cell-derived Leydig-like cell, which is obtained after the human pluripotent stem cell-derived Leydig-like cell of claim 11 is passaged at least once.

14. A human pluripotent stem cell-derived Leydig-like cell population, comprising the human pluripotent stem cell-derived Leydig-like cell of claim 11.

15. The human pluripotent stem cell-derived Leydig-like cell population according to claim 14, wherein the population comprises 80% or more of a Leydig-like cell capable of producing testosterone.

16. The human pluripotent stem cell-derived Leydig-like cell population according to claim 14 or 15, wherein the population is subjected to immuno-isolation treatment.

17. A pharmaceutical composition for treating a disease associated with a decrease in testosterone, comprising the human pluripotent stem cell-derived Leydig-like cell population of claim 14 or 15 as an active ingredient.

18. A composition for cell transplantation, comprising a biocompatible membrane to which the human pluripotent stem cell-derived Leydig-like cell population of claim 14 or 15 adheres.

19. A device for pluripotent stem cell-derived endocrine cell transplantation, comprising a biocompatible membrane for transplanting a pluripotent stem cell-derived endocrine cell into a living body.

20. The device for pluripotent stem cell-derived endocrine cell transplantation according to claim 19, wherein the pluripotent stem cell-derived endocrine cell is a human pluripotent stem cell-derived endocrine cell capable of producing testosterone.

21. The device for pluripotent stem cell-derived endocrine cell transplantation according to claim 20, wherein the human pluripotent stem cell-derived endocrine cell capable of producing testosterone is the human pluripotent stem cell-derived Leydig-like cell of claim 11.
